# EUROPÄISCHE PATENTSCHRIFT

(11) **EP 1 169 056 B1**
(45) Veröffentlichungstag und Bekanntmachung des Hinweises auf die Patenterteilung: **17.09.2003**
(21) Anmeldenummer: 00926813.7
(22) Anmeldetag: 05.04.2000
(51) Int. Cl.: A61K 38/55, A61K 38/07, A61K 38/08, A61P 37/06, A61K 38/16

(54) **CASPASE 8-INHIBITOREN ZUR IMMUNSUPPRESSION**
CASPASE-8 INHIBITORS FOR IMMUNOSUPPRESSION.
INHIBITEURS DE LA CASPASE-8 COMME IMMUNOSUPPRESSEURS

(30) Priorität: 06.04.1999 DE 19915465
(43) Veröffentlichungstag der Anmeldung: 09.01.2002
(73) Patentinhaber: Apoxis SA, 1066 Epalinges (CH)
(72) Erfinder: BUDD, Ralph, C., South Burlington, VT 05403 (US); TSCHOPP, Jürg, CH-1066 Epalinges (CH); KATAOKA, Takao, Tokyo 169-0074 (JP)
(74) Vertreter: Tetaz, Franck Claude Edouard
(86) Internationale Anmeldenummer: EP0003019
(87) Internationale Veröffentlichungsnummer: WO00059536

(56) Entgegenhaltungen:
- EP-A- 0 842 665
- WO-A-93/05071
- WO-A-96/25946
- WO-A-97/35876
- WO-A-98/31801
- WO-A-98/41232
- WO-A-98/57664
- US-A- 578 613
- US-A- 5 798 442
- US-A- 5 808 001
- DATABASE MEDLINE 200139552, Februar 2000 NOMURA Y. ET AL.
- DATABASE MEDLINE 1999439783, 04 Oktober 1999 DJERBI M. ET AL.

## Beschreibung

Die Erfindung betrifft die Verwendung eines Inhibitors von Cysteinaspartatprotease-8 (Caspase-8), der eine wichtige Funktion bei der intrazellulären Signaltransduktion zukommt, zur Herstellung eines Arzneimittels zur Proliferationshemmung von Zellen. Weiterhin betrifft die Erfindung die Verwendung eines solchen Inhibitors oder mehrerer solcher Inhibitoren zur Herstellung eines Arzneimittels zur Suppression der Immunantwort von Lymphozyten.

Aus der Literatur ist bekannt, daß Caspasen (Cysteinaspartatproteasen) für die intrazelluläre Signaltransduktion gewisser exogener Apoptose-stimulierender Signale von erheblicher Bedeutung sind. Bei der Apoptose handelt es sich um einen physiologisch fein regulierten zielgerichteten Zelltod, der beispielsweise durch Bindung von Liganden an Rezeptoren, z.B. TNP-Bindung oder CD95 (Fas)-Bindung, durch Entzug gewisser Wachstumsfaktoren, durch Ablösung von einer extrazellulären Matrix, durch ionisierende Strahlung, Staurosporin oder auch durch Glucokortikoide induziert werden kann. Aus der Literatur ist weiterhin bekannt, daß insbesondere nach einer durch extrazelluläres sFasL, also löslichem Liganden des Fas-Rezeptors, induzierten Oligomerisierung des Rezeptors Fas (CD 95) eine Kaskade von proteolytischen Reaktionen ausgelöst wird, die schließlich in die Zellapoptose einmündet. Hierbei wird entsprechend der Caspasenfolge in der Kaskade die jeweils folgende Caspase durch proteolytische Spaltung aktiviert. Eine zentrale Funktion bei dieser apoptotischen Signaltransduktion nimmt am Ausgangspunkt der proteolytischen Kaskade die sogenannte Caspase-8 ein, die zum DISC-Komplex gehört und sich über das Linker-Protein FADD an den Fas-Rezeptor anlagert. Am DISC-Komplex wird die enzymatisch noch inaktive Procaspase-8 in zwei aufeinanderfolgenden Reaktionsschritten gespalten, wodurch aktive Caspase-8 gebildet wird, die als Heterotetramer vom DISC-Komplex dissoziiert. Diese aktive Caspase-8 kann nunmehr beispielsweise die in der Kaskadenfolge weiter distal fungierenden Caspasen Caspase-3 oder Caspase-7 aktivieren.

Es ist weiterhin beschrieben, daß verschiedene Typen von Caspase-Inhibitoren existieren, die den apoptotischen Signaltransduktionsweg blockieren. Hierbei sind nicht biologisch auftretende Caspase-Inhibitoren von biologischen, beispielsweise viralen Caspase-Inhibitoren, zu unterscheiden.

In den Dokumenten WO-A-96/25946, WO-A-93/05071, WO-A-98/57664 und US-A-5,808,001 sind Inhibitoren verschiedener Caspasen beschrieben, zum Beispiel in der WO 93/05071 Inhibitoren der Caspase-1. Derartige Caspase-1-Inhibitoren werden auch in der WO 96/25946 offenbart, insbesondere der Caspase-1-Inhibitor YVAD. In der WO 98/57664 werden ebenfalls Caspase-1-Inhibitoren offenbart, und zwar im Hinblick auf die Verwendung bei Schädigungen des zentralen Nervensystems, insbesondere zur Behandlung der amyotrophen Lateralsklerose. In der US 5,808,001 werden sogenannte ICE-Inhibitoren offenbart, die wiederum die Caspase-1-Aktivität inhibieren. In der US 5,798,442 werden Peptidylderivate offenbart, die als Inhibitoren von Apopain (auch als CPP32 oder Caspase-3 bezeichnet), wie zum Bespiel Ac-DEVD-AMC, die zur Behandlung von Immundefizienz oder Diabetes eingesetzt werden können. Gemäß der Offenbarung der WO-A-97/35876 werden ICE-Inhibitoren (also Caspase-1-Inhibitoren) zur Behandlung von multipler Sklerose und anderen Erkrankungen, in denen die Apoptose von Neuronen für die Symptomatik ursächlich ist, beschrieben. Gemäß der Lehre der WO 98/41232 werden Zusammensetzungen beschrieben, die Corticosteroide und ein weiteres Agens das antagonistisch auf Zielsubstanzen wirkt, die die Produktion von Interferon-γ regulieren, enthalten. Bei dem Agens kann es sich um einen Inhibitor der Familie der Caspase handeln. Zusammensetzungen gemäß der WO 98/41232 können u.a. auch zur Behandlung von Patienten, die unter Gewebeabstoßung leiden, eingesetzt werden. Aus der EP 0842665 A2 ist bekannt, daß Inhibitoren verschiedener Interleukin-1β- konvertierender Enzyme (ICE) bspw. zur Behandlung von Alzheimer, rheumatoider Arthritis, Sepsis, MS, ALS, ischämischen Störungen, AIDS oder Autoimmunerkrankungen eingesetzt werden können.

Schließlich wird bei Kobayashi et al. (Arthritis & Rheumatismus, Vol. 42, 1999, S. 519 ff) dargelegt, daß Caspase-8-Inhibitoren für therapeutische Zwecke zur Behandlung von multipler Sklerose oder rheumatischer Arthritis eingesetzt werden können.

In der WO 98/11129 werden C-terminale modifizierte 2-Indolyldipeptide als Inhibitoren der ICE-Cysteinprotease-Familie offenbart, wobei bspw. neurodegenerative Erkrankungen und Autoimmunerkrankungen als therapeutische Einsatzgebiete genannt werden.

Der Literatur ist bisher ausschließlich zu entnehmen, daß Caspasen in Zusammenhang mit der apoptotischen Signaltransduktion Bedeutung zukommt. Folglich ist auch den Caspase-Inhibitoren, seien sie natürlichen oder nicht natürlichen Ursprungs, ausschließlich eine Funktion bei der Inhibition der Apoptose zugeordnet worden. Weitere funktionelle Eigenschaften der Caspasen im intrazellulären Stoffwechsel - und damit auch weitere Verwendungsmöglichkeiten für Caspase-Inhibitoren- sind dagegen im Stand der Technik nicht beschrieben.

Der vorliegenden Erfindung liegt nunmehr die Aufgabe zugrunde, für die Caspase-8 weitere funktionelle zellphysiologische Aktivitäten aufzufinden und damit neue Verwendungsmöglichkeiten für Caspase-8-Inhibitoren, auch unter Berücksichtigung pathophysiologischer Aspekte, zu bestimmen.

Die vorliegende Aufgabe wird erfindungsgemäß durch Anspruch 1, gelöst.

Gemäß Anspruch 1 wird die Verwendung eines Caspase-8-Inhibitors oder mehrerer Caspase-8-Inhibitoren zur Herstellung eines Arzneimittels zur Suppression des Immunsystems nach allogener Zell-, Gewebe- oder Organtransplantation beansprucht.

Nach Anspruch 2 kann ein Caspase-Inhibitor auch zur Inhibierung der Proliferation von Peripheren Blutlymphozyten (PBL) eingesetzt werden. Diese Verwendungsmöglichkeit beruht auf der Erkenntnis, daß Caspasen bspw. Caspase-8 nicht nur an der Weiterleitung exogener apoptotischer Signale beteiligt sind, sondern auch Funktionen bei der Proliferation von PBL nach entsprechender exogener Stimulierung übernehmen können. Dies läßt sich dadurch erklären, daß für die Proliferation von PBL zwei stimulierende Signale benötigt werden. Neben der seit langem bekannten stimulierenden Bindungsreaktion am T-Zellrezeptor-CD3 -Komplex muß nämlich ein weiteres costimulatorisches Signal vorliegen. Dieser Costimulus ist die Bindung von extrazellulärem FasL an Fas mit anschließender intrazellulärer Signaltransduktion durch die Aktivierung von Caspasen. Damit wirkt extrazelluläres PasL costimulatorisch beispielsweise neben der Stimulation von T-Zellen am T-Zell-Rezeptor-(TCR)/CD3-Komplex.

Also weisen Caspasen bspw. Caspase-8 eine Doppelfunktion auf, nämlich einerseits als Kaskadenglieder der apoptotischen Signaltransduktion und andererseits gemäß vorliegender Erfindung auch als intrazelluläre Elemente co-stimulatorischer exogener Signale für die PBL-Proliferation. Die Inhibition der Caspase-8 führt somit erfindungsgemäß auch zu einer Inhibition der PBL-Proliferation. Daher wirken Caspase-8-Inhibitoren gleichfalls als Inhibitoren der Proliferation von Lymphozyten und hierbei insbesondere von PBL. Caspase-8-Inhibitoren können also erfindungsgemäß die Proliferation von B- und insbesondere von T-Lymphozyten, vor allem bei im Blutkreislauf zirkulierenden Lymphozyten, inhibieren.

Dabei können die zur Proliferationshemmung eingesetzten Caspase-8-Inhibitoren ihre inhibitorische Wirkung durch reversible oder irreversible Inhibition der Caspase-8 ausüben. Auf diese Weise wird die intrazelluläre Signalübertragung blockiert.

Insbesondere sind solche Caspase-Inhibitoren Gegenstand der vorliegenden Erfindung, die die Funktion der Caspase-8 inhibieren. Die Caspase-8-Inhibition kann beispielsweise durch solche Substanzen bedingt sein, die die Abspaltung der Prodomäne der Procaspase-8 verhindern. Auf diese Weise kann keine aktive Caspase-8-Fraktion gebildet werden, die die weitere Signaltransduktion erlauben könnte. Es ist aber durch die Verwendung solcher Caspase-Inhibitoren denkbar, die die enzymatische Aktivität der proteolytisch gespalteten - und somit aktiven Caspase-8-Fraktion blockieren. Dies ist z.B. durch Bindung an das aktive Zentrum der Caspase-8 möglich.

Die Verwendung von Caspase-8-Inhibitoren ist aber auch zur Suppression einer überschießenden Immunantwort - sei es durch B-Lymphozyten, sei es durch T-Lymphozyten - angezeigt. Zu beachten ist hier insbesondere, durch den Einsatz von Caspase-8-Inhibitoren solche Erkrankungen, Störungen oder pathophysiologischen Zustände zu behandeln, bei denen sich die Immunabwehr gegen körpereigene Strukturen richtet.

Weiterhin ist die Verwendung eines Caspase-8-Inhibitors oder die kombinierte Verwendung mehrerer Caspase-8-Inhibitoren dann vorteilhaft, wenn eine grundsätzliche Suppression des Immunsystems erwünscht ist. Dabei sind Caspase-8-Inhibitoren insbesondere zur Suppression der durch Periphere Blutlymphozyten (PBL) getragenen Immunantwort geeignet. Eine umfassende Immunsuppression ist vor allem nach Transplantationen allogener Zellen, Gewebe oder Organe indiziert. Durch den Einsatz von Caspase-Inhibitoren bzw. deren Einsatz zur Herstellung eines Arzneimittels kann dergestalt die Abstoßungsreaktion des transplantierten Patienten gegen Fremdzellen, Fremdgewebe oder Fremdorgane unterbunden werden, ohne daß schwerwiegende Nebenreaktionen zu erwarten sind.

Bei den Caspase-8-Inhibitoren kann es sich um solche Substanzen handeln, die natürlich auftreten und ggf. physiologisch bereits als Caspase-8-Inhibitoren wirken. Es kann sich bei den Caspase-8-Inhibitoren aber beispielsweise auch um organisch-chemische Molekülstrukturen oder um kurze, nicht natürliche Peptide handeln. Allgemein sind also nicht biologisch auftretende, die Caspase-8 inhibierende Substanzen bzw. Moleküle bevorzugt.

Bevorzugt sind dabei Oligo- oder Polypeptide, die die Caspase-8 als Inhibitoren blockieren können. Oligopeptide mit 3 bis 15 Aminosäuren Kettenlänge sind besonders geeignet, ganz besonders bevorzugt sind solche mit 3 bis 6 Aminosäuren Kettenlänge,
wobei hierbei wiederum Tetrapeptide besonders vorteilhaft sind. Bei den Oligopeptiden kann es sich um Teilsequenzen natürlich auftretender und ggf. als Caspase-8-Inhibitoren wirkender Proteine handeln.

So können beispielsweise Teilsequenzen des bakteriellen Proteins CrmA eingesetzt werden. Derartige Caspase-8-Inhibitoren auf Peptidbasis können chemisch an reaktiven Gruppen der Aminosäure-Seitenketten, z.B. an Amino- oder Carboxygruppen, oder aber auch am jeweiligen N- bzw. C-Terminus des Peptids modifiziert sein. Derart kann beispielsweise die Stabilität des Inhibitors auf Peptidbasis erhöht oder die Passage des Inhibitors durch die Zellmembran erleichtert werden.

Als bevorzugte Modifizierungen am C-Terminus des Oligo- oder Polypeptids wären zu nennen: Aldehyd-Derivatisierung, die Einführung einer Fluoromethylketon- oder Acyloxymethylketongruppe.

Ganz besonders bevorzugt sind für die Verwendung als Caspase-Inhibitoren solche Peptide, die die Aminosäuresequenz IETD (Ein-Buchstabencode) enthalten. Aber auch das Peptid IETD, ggf. chemisch modifiziert, ist für die erfindungsgemäße Verwendung geeignet. Insbesondere bevorzugt als Caspase-Inhibitoren zum Einsatz als Proliferationshemmer ist IETD-fmk also jeweils durch eine Fluoromethylketongruppe am C-Terminus modifizierte Peptide.

Neben nicht natürlichen Molekülstrukturen zur Verwendung als Inhibitoren der Zellproliferation umfaßt die vorliegende Erfindung auch jene biologisch auftretenden Substanzen, insbesondere Peptide oder Proteine, die sich physiologisch als wirksame Caspase-8-Inhibitoren erweisen. Dabei kann es sich um Substanzen viralen, bakteriellen oder eukaryotischen Ursprungs handeln. Beispielhaft zu nennen wäre das bakterielle Protein CrmA.

Je nach Indikationsgebiet kann der Caspase-8-Inhibitor oder eine Kombination von zwei oder mehr Caspase-8-Inhibitoren in systemischer oder topischer Applikation eingesetzt werden. Bei systemischer Applikation kommen orale, intravenöse, intraperitoneale oder intramuskuläre Verabreichungsformen in Betracht.

Ggf. werden zur Verwendung des Caspase-8-Inhibitors zur Herstellung eines Arzneimittels zur Behandlung der zuvor genannten Erkrankungen oder Störungen Adjuvantien erforderlich sein. Die spezifische galenische Aufbereitung ist abhängig vom jeweiligen Indikationsgebiet und der gewünschten Verabreichungsform.

Die vorliegende Erfindung wird durch die nachfolgend beschriebenen Figuren näher erläutert:
Figur 1 stellt die Wirkung von verschiedenen Caspase-Inhibitoren auf humane T-Zellen dar. Hierzu wurden die T-Zellen mit 10 µg/ml löslichem anti-CD3-Antikörper in Gegenwart verschiedener Caspase-Inhibitoren stimuliert. Herangezogen wurden die Inhibitoren IETD-fmk und zVAD-fmk (Inhibitoren der Caspase-8), YVAD-fmk (Inhibitor der Caspase-1) sowie zur Kontrolle DMSO in jeweils vergleichbarer Konzentration. Aufgetragen findet sich die Proliferation der stimulierten T-Zellen (cpm, gemessen durch [³H]-Thymidin-Inkorporation) als Funktion steigender Konzentrationen der Caspase-Inhibitoren. Insbesondere die beiden Caspase-8-Inhibitoren IETD-fmk und zVAD-fmk zeigen bei Konzentrationen oberhalb von 25 µM eine deutliche Inhibitionswirkung auf die Zellproliferation.
Figur'2 stellt die Effekte dar, die sich bei Zugabe von Fas-Fc bzw. IgG für die Proliferation von T-Lymphozyten ergeben. Dabei wurden die T-Lymphozyten mit Hilfe von immobilisiertem anti-CD3-Antikörper aktiviert (0,5 µg/ml). Während die dosisabhängige Zugabe von Fas-Fc die nach Ablauf von drei Tagen gemessene Zellzahl (hier gemessen durch cpm) deutlich reduziert, hat die Zugabe von IgG keinerlei Wirkung auf die Zellproliferation. Das Ergebnis in Fig. 2 ist somit konsistent mit dem Modell, das postuliert, daß der immobilisierte FasL-Inhibitor Fas-Fc das für die Proliferation notwendige costimulierende Signal, nämlich die FasL/Fas-Bindung, blockiert.
Figur 3 gibt die spezifischen profilerationshemmenden Effekte von drei Caspase-Inhibitoren, nämlich YVAD-fmk, zVAD-fmk und IETD-fmk, wieder. Hierzu wurden PBL jeweils mit den zuvor bezeichneten Caspase-Inhibitoren kultiviert und dann mit 3 µm/ml löslichem anti-CD3-Antikörper und "crosslinked" sFasL stimuliert (50 ng/ml). "Crosslinked" sFasL stellt oligomerisiertes sFasL dar. Das sFasL trägt eine FLAG-Sequenz, an die kreuzweise vernetzende anti-FLAG-Antikörper binden, was zur Oligomerisierung führt. Ähnlich wie in Fig. 1 erweisen sich auch bei kombinierter Stimulierung mit anti-CD3-Antikörper und FasL die Caspase-8-Inhibitoren als die wirksamsten T-Zell-Proliferationshemmer.
In Figur 4 wird die Korrelation von IL-2 Expression und dem Einsatz von Caspase-Inhibitoren anhand eines Balkendiagramms deutlich. Es wird einerseits erkennbar, daß die kombinierte Stimulierung von anti-CD3-Antikörpern und FasL eine - gegenüber einer ausschließlich mit anti-CD3-Antikörpern durchgeführten Aktivierung (hier als Kontrolle bezeichnet) - signifikant erhöhte IL-2-Produktion zur Folge hat. Andererseits erweisen sich auch hier die Caspase-8-Inhibitoren IETD-fmk und zVAD-fmk bei anti-CD3-Antikörper-Stimulierung als besonders wirksam in bezug auf die Suppression der IL-2-Produktion. Das in Figur 4 dargestellte Ergebnis beruht auf Versuchen, bei denen 10⁶ PBL/ml mit immobilisiertem anti-CD3-Antikörper (3 µg/ml) und FasL (50 ng/ml) in An- oder Abwesenheit von den zuvor bezeichneten Caspase-Inhibitoren (50 µM) kultiviert wurden. Die Überstände wurden nach 24 Std. abgenommen und mit Hilfe eines CTLL-Bioassays auf ihre IL-2-Konzentration hin untersucht.
In Figur 5 wird gezeigt, daß die T-Zell-Proliferationshemmung von Caspase-8-Inhibitoren, hier am Beispiel von zVAD-fmk, durch die Zugabe von IL-2 aufgehoben werden kann. Dies bedeutet, daß die Aktivität von Caspase-8 für die IL-2-Produktion der T-Zellen wesentlich ist. Bei den der Figur 5 zugrundeliegenden Versuchen wurden PBL mit 10 µg/ml löslichem anti-CD3-Antikörper in der An- oder Abwesenheit von zVAD-fmk (50 µM) bzw. in der Anwesenheit von anti-CD3-Antikörper-zVAD-fmk und 500 U/ml rekombinantem humanen IL-2 aktiviert. Während erfindungsgemäß die Zugabe von zVAD-fmk eine gegenüber der Kontrolle deutliche Verringerung der Zellzahl zum Beobachtungszeitpunkt bewirkt, wird bei Zugabe von IL-2 die Zellproliferation dramatisch gesteigert.
Figur 6 stellt einen sogenannten Western-Blot dar. Humane T-Zellen wurden entweder ohne Stimulierung (Kontrolle) mit löslichem anti-CD3-Antikörper allein (3 µg/ml) oder mit anti-CD3-Antikörper und sFasL (50 ng/ml), das wie oben beschrieben kreuzweise über seine Flag-Sequenz vernetzt ist, kultiviert. Die Zellysate wurden nach den in Figur 6 angegebenen Zeitpunkten hinsichtlich der Expression von Procaspase-8 bzw. von Spaltprodukten der Procaspase-8 untersucht. Hierbei indiziert der schwarze Pfeil die Lage der enzymatisch inaktiven Procaspase-8 im Western-Blot, während der offene Pfeil das enzymatisch aktive, proteolytisch gespaltene 26 kDa-Fragment anzeigt. Aus Figur 6 ist deutlich erkennbar, daß vier Stunden nach Kultivierungsbeginn in den Zellysaten die größte Konzentration an aktiver Caspase-8 vorliegt. Hierbei zeigt der Versuchsansatz mit kombinierter Zellstimulation durch anti-CD3-Antikörper und FasL eine gegenüber der ausschließlich durch anti-CD3-Antikörper hervorgerufenen T-Zell-Stimulierung signifikant erhöhte aktive Caspase-8-Fraktion. In einem weiteren Versuchsansatz (Figur 6, Westem-Blot, rechts) wurden T-Zellen mit einer Kombination von anti-CD3-Antikörpern und FasL für die Dauer von 6 Stunden stimuliert, und zwar in Gegenwart von 50 µM des Caspase-8-Inhibitors IETD-fmk. Auf Grund der Wirkung des Caspase-8-Inhibitors wurde die Spaltung von Caspase-8 während der Stimulierung blockiert, eine 26 kDa-Fraktion ist im Western-Blot in diesem Fall nicht detektierbar.

Die vorliegende Erfindung wird durch die nachfolgenden Aueführungsbeispiele näher erläutert:

### 1. Ausführungsbeispiel

Um die Aktivität von Caspase-Inhibitoren als Inhibitoren-der Zellproliferation nachzuweisen, wurde deren Wirkung auf humane Periphere Blutlymphozyten untersucht.

Hierzu wurden diese durch eine Ficoll-Hypaque-Zentrifugation präpariert. Die Zellen (5x10⁴ Zellen pro "well") wurden dann auf 96-"well"-Platten in Anwesenheit verschiedener Caspase-Inhibitoren bzw. zur Kontrolle in deren Abwesenheit kultiviert. Die Konzentration der Caspase-Inhibitoren wurde in einem Bereich von 25 bis 100 µM variiert. Anschließend erfolgte eine Stimulierung der Zellen durch anti-CD3-Antikörper (TR66) bzw. durch eine kombinierte Stimulierung mit anti-CD3-Antikörper und löslichem rekombinaten FasL mit oder ohne anti-Flag-Sequenz-Antikörper (1 µg/ml).

Während der letzten 18 Stunden der viertägigen Kultivierung wurde die Zellproliferation gemessen. Meßgröße der Zellproliferation war die Inkorporation von [³H]Thymidin in die proliferierenden Zellen.

Die eingesetzten Caspase-Inhibitoren (YVAD-fmk, zVAD-fmk und IETD-fmk) waren Produkte von Bachem und von Enzyme System Products. Das rekombinate FasL stammte von Alexis.

Die eingesetzten Caspase-Inhibitoren bewirken im Falle von IETD-fmk bzw. zVAD-fmk eine vollständige Inhibition der durch die anti-CD3-Antikörper hervorgerufenen Stimulierung. Der Caspase-Inhibitor zVAD-fmk läßt eine partielle Inhibition der Zellproliferation erkennnen (Figur 1).

Auch die kombinierte Stimulierung der humanen Peripheren Lymphozyten durch anti-CD3-Antikörper und FasL führt zu einem ähnlichen Ergebnis in bezug auf die Aktivität der eingesetzten Caspase-Inhibitoren (Figur 3).

### 2. Ausführungsbeispiel

Durch ein weiteres Versuchssystem wurde die Inhibition der Proliferation in der Fraktion der T-Lymphozyten durch Caspase-Inhibitoren bestimmt.

Hierzu wurden T-Lymphozyten (10⁶/ml) 24 h mit immobilisiertem anti-CD3-Antikörper (3 µg/ml) mit oder ohne "cross-linked" FasL (50 ng/ml) stimuliert. Die Überstände von solchen Ansätzen mit einem Caspase-Inhibitor bzw. ohne Caspase-Inhibitor als Kontrolle wurden nach der Stimulationszeitraum abgenommen und deren jeweilige IL-2-Konzentration mittels eines CTLL-Bioassays bestimmt. Die Il-2-Konzentration in den Überständen spiegelt dabei die T-Zellproliferation wider.

Im Ergebnis zeigt sich, daß die Gegenwart von Caspase-Inhibitoren, insbesondere von zVAD-fmk und IETD-fmk, die T-Zellproliferation blockiert. In diesen Versuchsansätzen ist nur eine schwache IL-2-Aktivität zu beobachten (Figur 4). Zwei Versuchsansätze ohne Caspase-Inhibitor-Zugabe dienen als Maßstab für die Beurteilung der nach Stimulierung mit anti-CD3-Antikörper bzw. in Kombination mit FasL zu erwartenden IL-2-Produktion (Figur 4, Balken links bzw. rechts).

Durch Zugabe von exogenem rekombinanten IL-2 kann die Proliferationsinhibition in jenen Ansätzen mit Caspase-Inhibitor wieder aufgehoben werden. Eine gegenüber dem Kontrollansatz stark erhöhte [³H]Thymidin-Inkorporation kann in diesem Fall gemessen werden (Figur 5).

### 3. Ausführungsbeispiel:

In diesem Ausführungsbeispiel wurden ruhende humane T-Lymphozyten unter verschiedenen Bedingungen kultiviert: (i) als Kontrolle ohne Stimulierung, (ii) ausschließliche Stimulierung mit löslichem anti-CD3-Antikörper (3 µg/ml) oder (iii) kombinierte Stimulierung mit anti-CD3-Antikörper und sFasL (50 µg/ml, kreuzweise vernetzt über die FLAG-/Sequenz-"crosslinked").

Aus den verschiedenen Ansätzen wurden nach 0, 2, 4, 6 und 22 h Zellen entnommen und lysiert. Die Zellysate wurden durch die Western-Blot-Technik hinsichtlich ihrer Caspase-8-Aktivität (Procaspase-8, 55 kDa-Fraktion, bzw. proteolytisch gespaltene, aktive Caspase-8, 26 kDa-Fraktion) untersucht.

Eine Bande der aktiven 26 kDa-Fraktion erscheint nur nach Stimulierung mit anti-CD3-Antikörper bzw. intensiver nach kombinierter Stimulierung mit sFasL. 4 h nach Stimulierungsbeginn liegt die aktive 26 kDa-Caspase-8-Fraktion, die durch Proteolyse aus der 55 kDa-Fraktion entsteht, in ihrer höchsten Konzentration vor (Figur 6).

In einem weiteren Versuchsansatz wurden ruhende T-Lymphozyten in Gegenwart des Caspase-Inhibitors IETD-fmk (50 µM) mit anti-CDS-Antikörper und "cross-linked" sFasL stimuliert (s.o.) und 6 h nach Stimulierungsbeginn lysiert. Die Western-Blot-Auftragung läßt - im Gegensatz zum Versuchsansatz ohne Caspase-Inhibitor - keine 26 kDa-Bande erkennen (Figur 6, rechts). Dies weist nacht, daß die proteolytische Spaltung von Caspase-8 auch im Falle der Co-Stimulierung mit sFasL durch IETD-fmk wirksam blockiert wird.

## Patentansprüche

1. Verwendung eines Caspase-8-Inhibitors oder mehrerer Caspase-8-Inhibitoren zur Herstellung eines Arzneimittels zur Suppression des Immunsystems nach allogener Zell-, Gewebe- oder Organtransplantation.

2. Verwendung eines Caspase-8-Inhibitors oder mehrerer Caspase-8-Inhibitoren zur Herstellung eines Arzneimittels nach Anspruch 1, wobei die Immunsuppression durch Suppression der Immunantwort durch PBL entsteht.

3. Verwendung eines Caspase-Inhibitors gemäß einem der Ansprüche 1 oder 2, **dadurch gekennzeichnet, dass** er eine nicht biologisch auftretende Molekülstruktur aufweist.

4. Verwendung eines Caspase-Inhibitors gemäß Anspruch 3, **dadurch gekennzeichnet, dass** er ein Oligo-, insbesondere ein Tetrapeptid, oder ein Polypeptid ist.

5. Verwendung eines Caspase-Inhibitors gemäß Anspruch 3 oder 4, **dadurch gekennzeichnet, dass** er als Oligo- oder Polypeptid am N- oder C-Terminus eine Modifizierung aufweist.

6. Verwendung eines Caspase-Inhibitors gemäß Anspruch 5, **dadurch gekennzeichnet, dass** der C-Terminus des Oligooder Polypeptids eine Aldehyd-Derivatisierung, ein Fluoromethylketon oder ein Acyloxymethylketon aufweist.

7. Verwendung eines Caspase-Inhibitors gemäß einem der Ansprüche 3 bis 6, **dadurch gekennzeichnet, dass** er als Oligo- oder Polypeptid die Sequenz IETD enthält.

8. Verwendung eines Caspase-Inhibitors gemäß Anspruch 6 oder 7, **dadurch gekennzeichnet, dass** er IETD-fmk ist.

9. Verwendung eines Caspase-Inhibitors gemäß einem der Ansprüche 1 oder 2, **dadurch gekennzeichnet, dass** er biologischen Ursprungs ist.

10. Verwendung eines Caspase-Inhibitors gemäß Anspruch 9, **dadurch gekennzeichnet, dass** er viralen, bakteriellen oder eukaryotischen Ursprungs ist.

## Claims

1. Use of a caspase-8-inhibitor or of several caspase-8-inhibitors for the preparation of a medicament for the suppression of the immune system after allogenic cell, tissue or organ transplantation.

2. Use of a caspase-8-inhibitor or several caspase-inhibitors for the preparation of a medicament according to claim 1, whereby the immune suppression is caused by suppression of the immune response by PBL

3. Use of a caspase-inhibitor according to one of claims 1 or 2 **characterized in that** the caspase-inhibitor comprises a non biologically existing molecular structure.

4. Use of a caspase-inhibitor according to claim 3 **characterized in that** the caspase-inhibitor is an oligo-peptide, in particular a tetrapeptide or a polypeptide.

5. Use of a caspase-inhibitor according to claim 3 or 4 **characterized in that** the caspase-inhibitor as oligo- or polypeptide has a modification at the N- or C-terminus.

6. Use of a caspase-inhibitor according to claim 5 **characterized in that** the C-terminus of the oligo- or polypeptide has an aldehyde-derivatization, a fluoromethylketone or a acyloxymethylketone.

7. Use of a caspase-inhibitor according to one of claims 3 to 6 **characterized in that** the caspase-inhibitor as oligo- or polypeptide contains the sequence IETD.

8. Use of a caspase-inhibitor according to claim 6 or 7 **characterized in that** the caspase-inhibitor is IETD-fmk.

9. Use of a caspase-inhibitor according to claims 1 or 2 **characterized in that** the caspase-inhibitor is of biological origin.

10. Use of a caspase-iohibitor according to claim 9 **characterized in that** the caspase-inhibitor is of viral, bacterial or eukaryotic origin.

## Revendications

1. Utilisation d'un inhibiteur de caspase-8 ou de plusieurs inhibiteurs de caspase-8 pour la préparation d'un médicament pour la suppression du système immunitaire suivant une transplantation allogène de cellule, de tissu ou d'organe.

2. Utilisation d'un inhibiteur de caspase-8 ou de plusieurs inhibiteurs de caspase-8 selon la revendication 1, dans laquelle la suppression du système immunitaire résulte de la suppression de la réponse immunitaire par PBL.

3. Utilisation d'un inhibiteur de caspase-8 ou de plusieurs inhibiteurs de caspase-8 selon l'une des revendications 1 ou 2, **caractérisée en ce qu'**il débute par une structure moléculaire non biologique.

4. Utilisation d'un inhibiteur de caspase-8 ou de plusieurs inhibiteurs de caspase-8 selon la revendication 3, **caractérisée en ce qu'**il s'agit d'un oligopeptide, en particulier un tétrapeptide, ou d'un polypeptide.

5. Utilisation d'un inhibiteur de caspase-8 ou de plusieurs inhibiteurs de caspase-8 selon l'une des revendications 3 ou 4, **caractérisé en ce que** l'oligopeptide ou le polypeptide présente une modification N- ou C-terminale.

6. Utilisation d'un inhibiteur de caspase-8 ou de plusieurs inhibiteurs de caspase-8 selon la revendication 5, **caractérisée en ce que** l'extrémité C-terminale de l'oligopeptide ou du polypeptide présente un dérivé aldéhyde, une fluorométhyle cétone ou une acyloxyméthyle cétone.

7. Utilisation d'un inhibiteur de caspase-8 ou de plusieurs inhibiteurs de caspase-8 selon l'une des revendications 3 à 6, **caractérisée en ce que** l'oligopeptide ou le polypeptide contient la séquence IETD.

8. Utilisation d'un inhibiteur de caspase-8 ou de plusieurs inhibiteurs de caspase-8 selon la revendication 6 ou 7, **caractérisée en ce qu'**il s'agit du IETD-fmk.

9. Utilisation d'un inhibiteur de caspase-8 ou de plusieurs inhibiteurs de caspase-8 selon l'une des revendications 1 ou 2, **caractérisée en ce qu'**il est d'origine biologique.

10. Utilisation d'un inhibiteur de caspase-8 ou de plusieurs inhibiteurs de caspase-8 selon la revendication 9, **caractérisée en ce qu'**il est d'origine virale, bactérienne ou eucaryote.
